# EUROPEAN PATENT APPLICATION

(11) **EP 1 342 485 A1**
(43) Date of publication of application: **10.09.2003**
(21) Application number: 03000706.6
(22) Date of filing: 13.01.2003
(51) Int. Cl.: A61M 16/08, A61M 16/10

(54) **Disposable filter with water trap**

(30) Priority: 05.03.2002 SE 0200655
(71) Applicant: Siemens-Elema AB, 171 95 Solna (SE)
(72) Inventor: Östberg, Anders, 17155 Solna (SE)

(57) **Abstract**

A disposable filter (2) for filtering exhaled gas in a breathing apparatus, which filter (2) comprises a housing (4) having an inlet (6) and an outlet (8) and a filter unit (10) disposed in the housing (4). In order to increase the efficiency of the disposable filter (2) a water trap (12) is integrated with the housing (4), whereby water that condenses in the disposable filter (2) is collected in the water trap (12).

## Description

The present invention relates to a disposable filter according to the preamble of Claim 1.

Disposable filters, especially bacteria filters, are used in respiratory devices such as ventilators and anesthetic apparatus to filter exhaled gas from a patient. The filter prevents the contamination of the device and the surroundings. The disposable filter is changed at regular intervals, for example once a day.

Moisture is a problem with the use of such a filter for exhaled gas. The exhaled gas is saturated with moisture which condenses in the filter. The water affects the through flow area and thereby the effectiveness of the filter. The water also results in an increase in the pressure drop across the filter.

A known way to avoid the build up of condensation is to supply heat to the filter. This however demands that energy is supplied and that some form of heating component is built in or around the filter. Such solutions are on the whole expensive and impractical to implement for disposable filters.

Another way to reduce the problem of condensation is to dehumidify the gas before it reaches the filter, or to place the filter so that water does not pool in it.

Both of these solutions demand however the use of several other components which reduces the usability of the disposable filter for all the different situations where filtration of exhaled gas is desired.

An aim of the present invention is to produce a disposable filter which, being simple, cheap and practical, can be used for the filtration of exhaled gas without the above described disadvantages.

This aim is achieved according to the present invention by means of the disposable filter according to the preamble of Claim 1 being devised as is evident in the characterising portion of Claim 1.

A water trap integrated with the disposable filter results in the water that condenses in the filter being drained from the filter unit itself and thereby not affecting the through flow area and pressure drop so long as the water trap does not become full.

The water trap may be suitably constructed using a transparent material so that the condensed water is visible.

The volume of the water trap may be adapted to the life expectancy of the disposable filter using an estimation of the amount of water that under normal conditions can be expected to condense. It is even possible to provide disposable filters with different volumes of water trap.

To enable the water level in the water trap to be more easily read the water that condenses may be coloured. One way to colour the water that condenses is to cover the walls of the water trap with an indicator which reacts with the water and thereby colours it. Such an indicator can react to the pH of the water or to some other of its properties. Indicators of this kind are known in the field of chemistry. The indicator should, of course, be safe.

Another way to colour the water is by means of colour ampoules, tablets or the like.

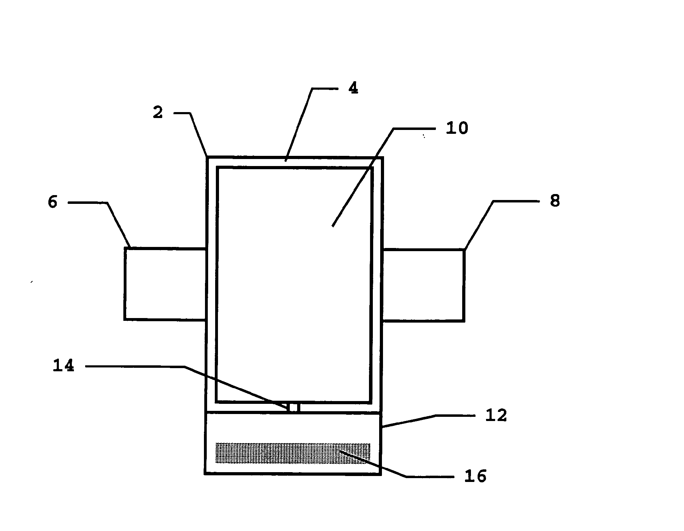

The figure shows an embodiment of the disposable filter according to the present invention.

An embodiment of the invention is shown in the form of a disposable filter 2. The disposable filter 2 comprises a housing 4 having an inlet 6 and an outlet 8 for connection of the disposable filter 2 to an expiratory part of a coupling to a ventilator or anesthetic apparatus (not shown in the figure).

The disposable filter 2 contains a filter unit 10 which filters the exhaled gas that passes through the disposable filter 2. The filter unit 10 may advantageously comprise a bacteria filter.

A water trap 12 is integrated with the housing 4 in order to collect the water that condenses in the filter unit 10 and the housing 4. The water is able to run into the water trap 12 via a connection 14. The connection 14 may be formed in a number of ways. It can comprise one or several holes, placed in different parts. It can comprise vents, a fine-meshed net, a water permeable filter or the like. The important thing is that water is able to pass to the water trap 12.

The simplest connection 14 comprises a hole that is equivalent to the whole contact surface. In principle this means that the housing 4 may be designed with a bigger volume than is occupied by the filter unit 10 and this excess volume forms the water trap 12. The filter unit 10 may be, for example, centred and/or kept in position by gluing against a wall of the housing 4. The water trap 12 is shown in the figure as being an asymmetrical part of the disposable filter 2 but it may even be symmetrically divided. The latter thus results in the disposable filter 2 being independent of placing.

The water trap 12 results in the filter unit 10 remaining effective during its entire expected lifetime without a decrease of the effective filter area and without a rise in the pressure drop over the disposable filter 2.

The volume of the water trap 12 may be adapted to a calculated maximum volume of condensed water during the life expectancy of the disposable filter 2.

The quantity of water becomes a measure of consumed time but, above all, the water in the water trap provides a clear indication that the disposable filter 2 is used.

To make it easier to see the water level, the condensed water can be coloured 16. This may be achieved with the aid of a colour ampoule or an indicator substance placed in the water trap 12.

## Claims

1. A disposable filter (2) for filtering exhaled gas in a breathing apparatus, which filter (2) comprises a housing (4) having an inlet (6) and an outlet (8) and a filter unit (10) disposed in the housing (4), **characterised in that** a water trap (12) is integrated with the housing (4), whereby water that condenses in the disposable filter (2) is collected in the water trap (12).

2. A disposable filter as claimed in claim 1, **characterised in that** a means for colouring (16) the water collected in the water trap (12) is provided.
